Europäisches Patentamt

European Patent Office

Office européen des brevets

(11) Veröffentlichungsnummer: **0 478 097 A1**

# EUROPÄISCHE PATENTANMELDUNG

(21) Anmeldenummer: **91250201.0**

(22) Anmeldetag: **18.07.91**

(51) Int. Cl.5: **C12N 11/04**

(30) Priorität: **24.08.90 DE 4027218**

(43) Veröffentlichungstag der Anmeldung:
**01.04.92 Patentblatt 92/14**

(84) Benannte Vertragsstaaten:
**DE DK FR GB IT NL**

(71) Anmelder: **Vorlop, Klaus-Dieter, Prof. Dr.**
**Hochstrasse 7**
**W-3300 Braunschweig(DE)**

(72) Erfinder: **Vorlop, Klaus-Dieter, Dr.**
**Hochstrasse 7**
**W-3300 Braunschweig(DE)**
Erfinder: **Remmers, Peter, Dipl.-Chem.**
**Lessingplatz 6**
**W-3300 Braunschweig(DE)**

(74) Vertreter: **Kaiser, Henning**
**Preussag AG, Patente und Lizenzen,**
**Postfach 15 12 27, Kurfürstendamm 32**
**W-1000 Berlin 15(DE)**

(54) **Verfahren zur Herstellung von Biokatalysatoren.**

(57) Ein Verfahren zur Herstellung von polymerfixierten Biokatalysatoren in Form von z. B. Perlen sieht vor, eine hochviskose Lösung aus einem Polyvinylalkohol mit geeigneten Mikroorganismen, Zellen oder Enzymen schockartig durch Eintropfen in eine tiefkalte Flüssigkeit wie Stickstoff zu Perlen zu formen und tiefzugefrieren und anschließend langsam, im Bereich von etwa -20 °C bis +5 °C mit nicht mehr als 4 °C/h aufzutauen, um eine stabile, elastische Gelbildung zu erreichen.

Der PVA-Lösung kann eine die Elastizität und Stabilität des Gels erhöhende Komponente wie Glyzerin zugesetzt werden. Weitere Zusätze können sein: kleine Glashohlkugeln zur Verminderung des Gewichts der Gelkörper, schwere Stoffe wie Titandioxid zur Erhöhung des Gewichts, Mineralien, Porenbildner und lichtundurchlässige Stoffe, die eine UV-Bestrahlung ohne Nachteil für die Mikroorganismen erlauben. Durch eine Zweistoffdüse lassen sich auch Biokatalysatoren mit einer äußeren, zellfreien Schicht aus PVA herstellen.

Die Erfindung betrifft ein Verfahren zur Herstellung von Mikroorganismen und Enzyme enthaltenden polymerfixierten Biokatalysatoren in Gestalt von aus einem Gel gebildeten, kleinen Körpern oder Perlen.

Im "Enzyme Technology, III. Rotenburg Fermentations Symposium 1982" Springer Verlag 1983 werden auf den Seiten 219 bis 235 von K.-D. Vorlop und J. Klein verschiedene Möglichkeiten zur Herstellung und Anwendung von in perlenförmigen Gelen eingeschlossenen Zellen als Biokatalysatoren beschrieben. Dabei werden z. B. Alginate und Chitosan als Gelbildner verwendet, deren hohe Kosten aber nur einen Einsatz bei hochqualifizierten Produkten gestatten. In DE-OS 34 32 923 wird zur Verbesserung der Lebensdauer derartiger Biokatalysatoren, die beispielsweise Hefezellen für die zweite Gärung bei der Sektherstellung enthalten, vorgeschlagen, auf die Oberfläche der Biokatalysatoren eine zellenfreie Schicht aus einem für Zellen undurchlässigen Gel zu bilden. In der Patentanmeldung P 38 36 894.3 wird ein Verfahren angegeben, gleichzeitig eine Vielzahl von Katalysatorperlen aus einer Zellen enthaltenden polymeren Lösung mittels z. B. Alginat oder Chitosan herzustellen.

Nach der EP-A1-0 303 122 lassen sich Biokatalysatoren mit immobilisierten Enzymen oder Mikroorganismen in einem Gel hoher mechanischer Festigkeit aus einem Polyvinylalkohol hohen Polymerisationsgrades dadurch herstellen, daß eine wässrige Lösung gebildet wird, die Polyvinylalkohol, Mikroorganismen und ein wasserlösliches, gelbildendes Polysacharid wie Alginat enthält, daß diese Lösung in eine Metallionen enthaltende wässrige Lösung eingetropft wird und daß die hier gebildeten Körper bei einer Temperatur unter -5° C in gegebenenfalls mehreren Zyklen eingefroren und wieder aufgetaut werden.

Die Auswaschung von Polyvinylalkohol aus Perlen, die in ähnlicher Weise hergestellt wurden, konnte nachgewiesen werden. Ein weiterer Nachteil der bekannten Verfahren ist, daß aus den zunächst gebildeten Ca-Alginat-Perlen ein großer Teil des Polyvinylalkohols in die Vernetzerlösung diffundiert, diese Lösung verschmutzt und die für die Gelfestigkeit wichtige Konzentration von Polyvinylalkohol in den Perlen verringert.

In EP-A1-0 060 052 wird ein Verfahren zur Immobilisierung lebender Mikroorganismen in aus Polyvinylalkohol gebildeten Gelen beschrieben. Es wird eine wässrige Lösung mit 1 bis 25 Gew.-% Polyvinylalkohol mit einem Hydrolysierungsgrad von mindestens 95% und einem Polyinerisationsgrad von nicht weniger als 1.500 hergestellt. Dieser Lösung, die noch zusätzlich Mineralien enthalten kann, werden die Mikroorganismen zugegeben. Die Lösung wird in Formen zu Körpern oder Folien gewünschter Gestalt bei einer Temperatur unter -6° C eingefroren und die Körper werden in gefrorenem Zustand bei Unterdruck dehydriert, wodurch die Bildung von elastischen Gelen bewirkt wird, und danach aufgetaut. Dieses Verfahren mag zweckmäßig sein, um Körper von besonderer Gestalt zu erhalten, es ist aber für etwa kugelförmige Gel-Körper und wegen der Anwendung von formenden Gefriereinrichtungen und des Entwässerns bei Gefriertemperatur und Vakuum sehr aufwendig.

Der Erfindung liegt die Aufgabe zugrunde, die Herstellung von polymerfixierten Biokatalysatoren in Form von Mikroorganismen und Enzyme immobilisierenden perlenförmigen Körpern in der Weise zu verbessern, daß die Herstellung vereinfacht und beschleunigt wird und Körper hohe Elastizität, Abriebfestigkeit und Verwendungsdauer entstehen.

Diese Aufgabe wird mit dem in Anspruch 1 beschriebenen Verfahren gelöst. Die Unteransprüche geben bevorzugte Ausgestaltungen des Verfahrens an.

Danach wird zunächst eine wässrige Lösung mit einem Verhältnis von 40 bis 150 g Polyvinylalkohol (PVA) zu 1 l Wasser, vorzugsweise 70 bis 100 g/l aus einem Polyvinylalkohol mit einem Polymerisationsgrad von über 1.200 vorzugsweise von über 1.500 bis etwa 1.700 und einem Hydrolysegrad von mindestens 98% hergestellt, und dieser hochviskosen Lösung werden die als Biokatalysatoren wirksamen biologischen Substanzen (Zellen oder Enzyme) in einer Menge von bis zu 400 g je 1 l Lösung zugemischt. Der Zusatz eines Alginates oder eines anderen Gelbildners ist nicht erforderlich, wodurch die Herstellungskosten reduziert werden. Die Lösung wird in Tropfenform schockartig tiefgefroren auf eine Temperatur von unter -30° C und vorzugsweise darunter. Besonders bevorzugt erfolgt das Schockgefrieren auf eine Temperatur unter -190° C, wozu die Lösung in verflüssigte Gase wie Luft oder Stickstoff eingetropft wird. Es wurde gefunden, daß bei diesem schockartigen Tiefgefrieren die Mikroorganismen nicht geschädigt wurden, da es eine wesentliche Kristallisation verhindert. Das Eintropfen in solche tiefkalten Flüssigkeiten mit niedrigem Siedepunkt reicht für die Formgebung der Perlen aus und erspart ein anschließendes Waschen der Biokatalysatoren.

Zwar tritt bei Polyvinylalkohol eine Gelbildung schon bei Abkühlung auf -18 bis -20° C mit anschließendem Auftauen auf, aber ein Einfrieren auf diese Temperaturen, insbesondere wenn es langsam erfolgt, schädigt die Zellen und ist mit zwischenzeitlichem Auftauen mehrfach zu wiederholen, um aus dem Gel genügend feste Körper, Kugeln oder Perlen zu erhalten. Durch Schockgefrieren von Tropfen in einem sehr kalten Medium bei tiefen Temperaturen werden dagegen sehr elastische Körper mit fester Oberfläche erzeugt, ohne daß die eingeschlossenen Mikroorganismen nachteilig beeinflußt werden.

Die Aufenthaltsdauer der tiefgefrorenen Körper in dem tiefkalten Medium kann so kurz wie praktisch möglich sein, da die Tropfen und gefrorenen Perlen einen je nach dem späteren Verwendungszweck zwischen etwa 0,5 und 4 mm liegenden Durchmesser aufweisen und der Kälteübergang ins Innere daher sehr schnell bei den tiefen Temperaturen erfolgt.

Es wurde weiter gefunden, daß für die Ausbildung stabiler Gel-Körper das Auftauen langsam, schonend und kontinuierlich vorteilhaft ist. Eine Erwärmungsrate von bis zu 20 °C/h vorzugsweise von bis 4° C/h in dem Temperaturbereich von -30° C bis +15° C vorzugsweise -5 °C bis +5 °C wurde als optimal ermittelt. Werden die Körper in flüssigem Stickstoff bei unter -190° C eingefroren, so kann das Auftauen anfangs mit einer höheren Auftaugeschwindigkeit erfolgen, jedoch soll wenigstens im Temperaturbereich von -5 °C bis +5° C die Erwärmungsgeschwindigkeit von etwa 4 °C/h nicht überschritten werden. Ein wesentlich schnelleres Auftauen in diesem Bereich führte zu einer unbefriedigenden Ausbildung und Stabilität des aus dem Polyvinylalkohol gebildeten Gels. Eine Dehydratisierung ist bei dem genannten langsamen Auftauen zur Gelbildung nicht notwendig.

Das Auftauen erfolgt zweckmäßigerweise in einem gekühlten Raum, beispielsweise in einem Kühlschrank, einer Gefriertruhe und/oder einem wärmeisolierten Behälter (Dewargefäß), bei denen infolge geeigneter Vorkühlung, Isolierung und gegebenenfalls Wärmeabfuhr die oben genannte Erwärmungsrate eingehalten werden kann. Beim Auftauen von einer Temperatur von etwa -190° C wird wenigstens anfänglich verdunstender, flüssiger Stickstoff in den gekühlten Raum eingebracht werden.

Ein Zusatz von bis zu 40 Gew.-% Glyzerin oder Zucker, vorzugsweise bis zu 20 Gew.% als weichmachende Komponente erhöht die Stabilität und Elastizität der hergestellten Körper. Als Zusatz kommen auch hochsiedende, wasserlösliche organische Verbindungen mit Hydroxylgruppen wie z. B. Ethylenglykol in Frage.

Die Zusätze können gleichzeitig als Nährstoffquelle für die lebenden Mikroorganismen dienen.

Die hergestellten Körper oder Perlen besitzen eine glatte, abriebfeste Oberfläche, sind elastisch deformierbar, lassen sich aber zwischen den Fingern nicht zerdrücken, so daß sie auch eine für vorhersehbare Anwendungen gute Festigkeit aufweisen. Sie sind so elastisch, daß sie auch bei starker Bildung von Kohlendioxid im Inneren nicht aufplatzen. Ferner werden sie praktisch nicht biologisch abgebaut oder von Salzen angegriffen. Die Herstellung durch Bildung einer wässrigen Lösung, die Formgebung durch Tropfenbildung und durch schockartiges Tiefgefrieren der Tropfen in einer tiefkalten Flüssigkeit zu diskreten Kugeln und durch kontrolliertes langsames Auftauen, insbesondere in dem für die Gelbildung kritischen Temperaturbereich ist sehr einfach und daher auch für größere Mengen von Körpern möglich. Die hergestellten Biokatalysatoren sind im gleichen Maße aktiv wie solche, die z. B. mittels Alginat oder ähnlich gebildet werden. Im Gegensatz zu Alginat, Chitosan, Carragenan und ähnlichen gelbildenden biopolymeren Stoffen ist ein Gel aus Polyvinylalkohol biologisch schwerstabbaubar und wird auch nicht in Medien angegriffen, die Salze enthalten.

Die Formgebung beziehungsweise das Einfrieren ist auch in tiefkaltem Öl möglich, jedoch ist in diesem Fall damit zu rechnen, daß etwas Öl in die Kugel aufgenommen wird, was meist unerwünscht ist. Bevorzugt wird daher flüssiger Stickstoff, der für die Kugel keinen Nachteil hat, von ihrer Oberfläche schnell verdampft und bei dem ein Waschen der Kugeln nicht notwendig ist.

Die erfindungsgemäß hergestellten Biokatalysatoren können je nach den eingeschlossenen Mikroorganismen oder Enzymen in vielfältiger Weise eingesetzt werden. Beispiele sind die Entfernung von Ammonium, Nitrat und Pestiziden aus Trinkwasser, die Beseitigung von Ammonium und Nitrat sowie von Giftstoffen wie Phenol, Lösungsmitteln und Farbstoffen, soweit biologisch abbaubar in kleineren Kläranlagen. Weitere Anwendungen sind biologische Verfahren wie die Herstellung von Bioalkohol aus Zucker, Bierherstellung, Herstellung von Milchsäuren und Aminosäuren aus Zucker und anderen Rohstoffen und pharmazeutische Verfahren.

Das erfindungsgemäße Verfahren kann noch in verschiedener Weise weiter ausgestaltet werden. So können beispielsweise besonders leichte Perlen dadurch hergestellt werden, daß der Lösung aus Polyvinylalkohol und Mikroorganismen oder Enzymen etwa 5 bis 10 Gew.-% kleine Glashohlkugeln mit einem Durchmesser von 20 bis 100 μm zugefügt werden. Die so erzeugten Perlen schwimmen in einem Reaktor, indem sie durch Rühren oder Wirbelbildung mit einer zu behandelnden Flüssigkeit vermischt werden, leicht auf und lassen sich einfach abtrennen. Besonders schwere Perlen lassen sich durch einen Zusatz von wasserunlöslichen, die Zellen nicht beeinflussenden schweren Stoffen wie Titandioxid oder Bariumsulfat erzeugen. Perlen, deren Dichte größer als 1 ist, können beispielsweise auf einem von unten durchströmenden Bett eingesetzt werden.

Als Zusätze zu den Perlen können auch vorzugsweise poröse Minerale verwendet werden. In diesem Falle wird vorzugsweise eine Lösung mit den Zellen mit den Mineralen vermischt, so daß die Zellösung eindringt und danach die Lösung mit Polyvinylalkohol gebildet. Die Minerale können zusätzliche Hohlräume für die Zellen schaffen, die bewachsen werden können.

Ein anderer möglicher Zusatz ist der eines Stoffes, der die Lichtdurchlässigkeit der Perlen weitgehend ausschließt. Ein solcher Stoff ist Ruß. Lichtundurchlässige Perlen können nachträglich mit UV-Licht bestrahlt werden, um an der Oberfläche haftende Fremdbakterien abzutöten, ohne daß die im Inneren enthaltenen Zellen geschädigt werden.

Es kann der Lösung für die Perlen auch feinteilige Aktivkohle zugesetzt werden. Insbesondere bei der Behandlung von Flüssigkeiten, die Pestizide oder Herbizide enthalten, wirkt Aktivkohle als Adsorbens. In den Perlen stellt die Aktivkohle einen Langzeitspeicher für die Nährstoffe der Mikroorganismen dar, die auf diesem Speicher zurückgreifen, wenn vorübergehend keine neuen Schadstoffe herangeführt werden.

Obwohl in den meisten der vorher genannten Anwendungsfällen die zu behandelnden Schadstoffe in die Perlen eindringen und die Stoffwechselprodukte der Mikroorganismen austreten können, kann es in Einzelfällen zweckmäßig sein, Porenbildner einzusetzen. Durch die Poren wird eine bessere Zugänglichkeit zum Inneren der Perlen geschaffen, wobei die Poren jedoch so klein sein sollten, daß fremde Bakterien nicht in die Zellen eindringen. Mit den Poren in der Perlenoberfläche entstehen auch Poren oder Hohlräume im Inneren, die für das Wachstum der eingeschlossenen Zellen oder Organismen vorteilhaft sein können. Als Porenbilder werden der Lösung Stoffe zugesetzt, die sich langsam auflösen oder die zugleich Nährstoffe sind. Als Porenbilder können je nach der Anwendung der Perlen feingemahlene feste Stoffe wie Calciumkarbonat, Calciumsilikat, Calciumphospat, Magnesiumphosphat, Polyhydroxy-Buttersäure usw. sowie als flüssige Stoffe biologisch abbaubare Fette in feiner Verteilung oder kleine hydrophobe Öltröpfchen verwendet werden.

Schließlich können auch Stoffe zugesetzt werden, die Sauerstoff speichern oder deren chemische Konstitution gewährleistet, daß bei Zerfall Sauerstoff freigesetzt wird.

Die Bildung der Tropfen erfolgt unter Berücksichtigung der Viskosität der Lösung durch eine Düse, wobei die Größe der Tropfen veränderbar ist. Normalerweise ist eine Vielzahl paralleler Düsen oder oder Austrittsöffnungen vorhanden. Es ist aber auch möglich, Zweistoffdüsen zu verwenden, die eine zentrale Düsenöffnung besitzen, die von einer ringförmigen Düse für ein zweites Medium umgeben ist. Mit derartigen Düsen lassen sich Tropfen und somit auch Perlen herstellen, bei denen ein Kern aus einem ersten Medium von einer Hülle aus einem zweiten Medium bedeckt ist. So kann der Kern beispielsweise aus einer Lösung von Polyvinylalkohol mit Zellen gebildet werden und die Hülle besteht nur aus Polyvinylalkohol beziehungsweise dem hieraus gebildeten Gel. Es gibt auch die Möglichkeit, nur die Hülle als Gel aus Polyvinylalkohol auszubilden, wobei dann der Kern aus Zellen, Wasser und gegebenenfalls einem die Viskosität des flüssigen Kerns beeinflussenden Mittel besteht. Der zellenartige Kern kann beispielsweise einen Zusatz von Ruß enthalten und daher lichtundurchlässig sein, um an und in der Hülle vorhandene fremde Zellen durch UV-Bestrahlung abzutöten.

Beispiel 1

Eine Suspension aus Leitungswasser und 8 bis 10 Gew.% Polyvinylalkohol wird bei ca. 80 °C unter Rühren gelöst oder durch 10minütiges Autoklavieren bei 121 °C in Lösung gebracht. Die klare Lösung wird mit 10 Gew.% aufkonzentrierter Zellsuspension vermengt. Diese Suspension wird bei 30 bis 40 °C aus einer Abtropfeinrichtung in flüssigen Stickstoff getropft. Die so erhaltenen diskreten Perlen werden in einem Dewargefäß, in dem sie über längere Zeit verbleiben, langsam aufgetaut mit einer durchschnittlichen Auftaugeschwindigkeit von 4 bis 5 °C pro Stunde bis auf etwa Zimmertemperatur.

Die erhaltenen perlenförmigen Biokatalysatoren wiesen eine große Beständigkeit und eine hohe mechanische Festigkeit auf und konnten direkt eingesetzt werden.

Beispiel 2

Der Lösung gemäß Beispiel 1 wurden zusätzlich 20 bis 40 Gew.% Glyzerin oder Saccharose als eine Komponente, die die Gelstabilität erhöht und zum Schutz der Mikroorganismen vor Gefrierschäden beitragen kann, zugefügt. Die Festigkeit und Elastizität der Perlen konnte hierdurch noch verbessert werden.

Durch Variation der Abtropfeinrichtung wurde der Durchmesser der Perlen zwischen 2 und 5 mm verändert.

Beispiel 3
Einsatz von PVA-Biokatalysatorperlen zur Denitrifikation von nitratbelastetem Wasser

Die wie oben beschrieben dargestellten PVA-Biokatalysatoren werden für die kontinuierliche heterotrophe Denitrifikation in einem Rührreaktor eingesetzt. Die Nitratbelastung des Zulaufs beträgt 100 mg $NO_3^-$/l = 1,6 mmol $NO_3^-$/l. Das nitratbelastete Wasser wird aus Leitungswasser geringer Härte (2-3) und den entsprechenden Mengen $KNO_3$ (0,163 g/l) aufbereitet. Die C-Quelle wird über die pH-Regelung durch die Zudosage einer Essigsäure/Na Acetatlösung bereitgestellt. Dabei ist der pH-Wert der Säurevorlage durch gleichionigen Zusatz ($CH_2COO^-$) so eingestellt (ph-Wert = 4,8), daß die zudosierte Acetatmenge ausreicht, um das eingetragene Nitrat vollständig zu reduzieren. Der Rührreaktor wird mit 1,0 l Nitratwasser und 650 $cm^3$ (Schüttvolumen) PVA-Biokatalysatoren beschickt. Das Volumen des umgepumpten Wirbelbetts (Rotordrehzahl: 300 rpm) wird durch einen Überlauf, durch welchen auch die sich bildenden Gase entweichen, auf ca. 1,6 l gehalten. Die Reaktorinnentemperatur wird auf 20 °C eingestellt und gehalten. Zum Anfahren des Reaktors wird dieser zunächst ohne Zulauf von nitratbelastetem Wasser und mit einem Zusatz von Na Acetat betrieben. Die mit ca. 1 Gew.% BFM beladenen Biokatalysatorperlen zeigen eine Anfangsabbauaktivität von 0,04 mg $NO_3^-$/g Kat   h.

Nach viermonatigem Verbleib im beschriebenen Reaktorsystem zeigen die PVA-Biokatalysatorperlen keine mechanischen Verschleißerscheinungen.

Beispiel 4
Ethanolfermentation mit in Polyvinylalkohol immobilisierter Sacharomyces cerevisiae (Bäckerhefe)

In einem 300 ml fassendem Doppelmantelglasreaktor werden zum Vergleich der Anfangs- und Absolutaktivitäten, mit in unterschiedlichen Matrices eingeschlossener Bäckerhefe, Ethanolfermentationen durchgeführt. Die Messungen sollen in erster Linie Vergleichsdaten liefern, um die Effektivität der verschiedenen Immobilisierungsmethoden einordnen zu können. Bei Variation der Immobilisierungsmethode werden alle weiteren Fermentationsparameter konstant gehalten. Die Ethanolfermentation wird unter folgenden Bedingungen durchgeführt:
10 g eingesetzter Biokatalysator ist jeweils mit 10 Gew.% Bäckerhefe (DAW-Hefe Vital Gold Hamburg) beladen. Die immobilisierte Hefe wird bei 30 °C unter anaeroben Bedingungen in folgendem Medium inkubiert: Hefeextrakt 3,0 g/l, Malzextrakt 3,0 g/l, Pepton 5,0 g/l, Glukose 100 g/l.

Die Anfangsaktivität der unterschiedlich in Alginatvollkugeln und in PVA-Katalysatorperlen immobilisierten Bäckerhefe ist unter den gegebenen Bedingungen sehr ähnlich und im Vergleich mit freien, nicht immobilisierten Zellen unwesentlich kleiner. Die relativ hohe Anfangsaktivität und die Zunahme der Biomasse (Auswachsen der Zellen) zeigt, daß das Einfrieren nur einen geringen Aktivitätsverlust der Bäckerhefe mit sich bringt. Nach einer Reaktionszeit von etwa 1000 min. steigt die prozentuale Ethanolkonzentration bei der Behandlung mit PVA-Perlen noch an, während bei Alginatkugeln fast keine Zunahme mehr zu erkennen ist.

Beispiel 5
Verhalten der PVA-Katalysatormatrix unter Druckbelastung

Die bei der Verformung einer Katalysatorperle auftretenden Kräfte wurden ermittelt. Dabei wird das Verhalten einer PVA-Matrix bei der Be- und Entlastung einer Katalysatorperle mit einer klassischen Alginatperle verglichen.

Zur Bestimmung der Druckkräfte bei der mechanischen Verformung einer Katalysatorperle wird eine Testperle auf eine Auflageplatte gelegt. Dann wird eine Kraftmeßdose mit einer Geschwindigkeit von 1,5 mm/min abwärts bewegt. Das der jeweiligen Druckkraft entsprechende Meßsignal wird auf einen Schreiber gegeben, der die Kraft über der Zeit registriert. Durch den sich senkenden Stempel wird auf die Perle eine - bedingt durch den Vorschub - kontinuierlich wachsende Kraft ausgeübt. Perlen von 3 mm Durchmesser werden auf 0,3 mm zusammengedrückt.
Untersucht wurden Alginatperlen aus 3,5 %iger Na-Alginatlösung in 2 %iger $CaCl_2$-Lösung und PVA-Perlen aus 7 % PVA mit Zusatz von 15 % Saccharose, letztere gebildet in flüssigem Stickstoff.

Aus den erstellen Belastungsdiagrammen lassen sich nicht nur Aussagen über die Kraftaufnahme und Bruchfestigkeit der Katalysatorperlen machen, es können auch Daten über die irreversiblen und reversiblen Anteile der Verformung gewonnen werden. Die Verformung der Alginatperle auf ca. 1/10 ihres Durchmessers erfordert mehr Kraft, als bei der Verformung einer PVA-Perle aufgewand werden muß. Der steile Abfall der Kurve während der Entspannungsphase spricht allerdings gegen die Existenz von Rückstellkräften innerhalb der Alginatperle. Dies deutet auf die irreversible plastische Verformung der Alginatmatrix hin. Nach jedem Versuch bleibt eine "Alginattablette" zurück. Im Gegensatz dazu ist die PVA-Matrix ein Beispiel für elastische Polymernetzwerke. Der Kurvenverlauf während der Entlastungsphase entspricht fast spiegelbildlich dem der Belastungsphase. Die Kraftaufnahme der PVA-Perle bei der Kompression ist etwas geringer als beim Alginat. Dafür bildet sich nach der Entlastung eine nur geringfügig verformte Perle zurück, welche abermals vermessen werden kann. Dies ist ein quantitativer Beweis für die hohe Elastizität der PVA-Biokatalysatorperlen, welche damit für den Einsatz in bewegten Medien besonders gut geeignet sind.

Beispiel 6
Herstellung von Perlen mit äußerer PVA-Schutzschicht

Als Ausgangslösung für den zellhaltigen Kern wird eine 10%ige Hefelösung eingesetzt. Die äußere Schutzschicht wird aus einer zellfreien, 8%igen PVA-Lösung gebildet. Es wird eine Zweistoffdüse verwendet, durch deren inneren Teil die Hefesuspension und durch deren äußeren Teil die PVA-Lösung gepumpt werden, so daß einzelne umhüllte Tropfen in flüssigen Stickstoff fallen. Der flüssige Stickstoff wird abgetrennt, und die Perlen werden langsam aufgetaut, wobei wenigstens im Temperaturbereich von -20 °C bis 5 °C, vorzugsweise wenigstens im Bereich von -5 °C bis +5 °C eine Auftaugeschwindigkeit von 4 °C/h nicht überschritten wird. Die erhaltenen Biokatalysator-Hohlkugeln mit PVA-Hülle können anschließend z. B. zur Herstellung von Ethanol aus Glukose eingesetzt werden.

**Patentansprüche**

1. Verfahren zur Herstellung von Mikroorganismen und Enzyme enthaltenden polymerfixierten, perlenförmigen Biokatalysatoren aus einer Polyvinylalkohol von einem Polymisierungsgrad über 1.200 enthaltenden wässrigen Lösung durch Gefrieren und anschließendes Auftauen der aus Tropfen gebildeten Körper, dadurch gekennzeichnet, daß
   a) eine hochviskose Lösung im Verhältnis von 40 bis 150 g Polyvinylalkohol mit einem Hydrolysegrad von mindestens 98 % zu 1 l Wasser hergestellt wird,
   b) dieser Lösung bis zu 400 g je 1 l lebende oder tote Mikroorganismen, Zellen oder Enzyme beigemischt werden,
   c) die Lösung unter schockartigem Tiefgefrieren in ein unter -30° C kaltes, flüssiges Medium eingetropft wird, wobei gefrorene perlenförmige Körper entstehen,
   d) die gefrorenen Körper von dem kalten Medium getrennt werden und
   e) die gefrorenen Körper langsam und schonend in dem Temperaturbereich von -20° C bis 5° C mit einer Erwärmungsrate von bis zu 20 °C pro Stunde unter Ausbildung des Gels auftaut und erwärmt.

2. Verfahren nach Anspruch 1, dadurch gekennzeichnet, daß die Erwärmungsrate wenigstens im Temperaturbereich von -5 °C bis zu +5 °C einen Wert von 4 °C pro Stunde nicht überschreitet.

3. Verfahren nach Anspruch 1 oder 2, dadurch gekennzeichnet, daß man der hochviskosen Polyvinylalkohol-Lösung bis zu 40 Gew.-% eine weichmachende, die Elastizität des Gels steigernde Komponente zusetzt.

4. Verfahren nach einem oder mehreren der Ansprüche 1 bis 3, dadurch gekennzeichnet, daß als kaltes Medium, in das die Lösung eingetropft wird, ein flüssiges Gas wie Luft oder Stickstoff eingesetzt wird, daß ein Tiefgefrieren der Körper auf unter -190° C bewirkt.

5. Verfahren nach einem oder mehreren der Ansprüche 1 bis 4, dadurch gekennzeichnet, daß durch Zusätze zu der Lösung die Dichte der hergestellten Körper beeinflußt wird.

6. Verfahren nach Anspruch 5, dadurch gekennzeichnet, daß der Lösung 5 bis 10 Gew.-% kleine Glashohlkugeln von 20 bis 100 µm Durchmesser zugesetzt werden.

7. Verfahren nach Anspruch 5, dadurch gekennzeichnet, daß der Lösung schwere Stoffe wie Titandioxid und Boriumsulfat zur Erzeugung von Perlen mit einer Dichte größer als 1 zugefügt werden.

8. Verfahren nach einem oder mehreren der Ansprüche 1 bis 4, dadurch gekennzeichnet, daß poröse Mineralien und/oder Zeolithe zunächst mit einer die Zellen enthaltenden Lösung durchtränkt und anschließend der Lösung aus Polyvinylalkohol zugefügt werden.

9. Verfahren nach einem oder mehreren der Ansprüche 1 bis 4, dadurch gekennzeichnet, daß der Lösung Porenbildner in Form von sich langsam zersetzenden oder von den immobilisierten Zellen abbaubaren Stoffen zugesetzt werden.

10. Verfahren nach einem oder mehreren der Ansprüche 1 bis 9, dadurch gekennzeichnet, daß die Tropfen durch eine Zweistoffdüse gebildet werden, durch deren zentrale Öffnung eine die Zellen enthaltende Lösung austritt, die den Kern der Perlen bildet, und durch deren ringförmige äußere Öffnung eine Polyvinylalkohol enthaltende Lösung, die als Gel eine Hülle um den Kern bildet, austreten.

11. Verfahren nach einem oder mehreren der Ansprüche 1 bis 10, dadurch gekennzeichnet, daß der Lösung ein eine weitgehende Lichtundurchlässigkeit der Perlen bzw. Zellen enthaltende Perlenteil bewirkender Stoff wie Ruß zugesetzt wird.

12. Verfahren nach Anspruch 11, dadurch gekennzeichnet, daß die lichtundurchlässigen Perlen zur Abtötung fremder Bakterien auf ihrer Oberfläche mit UV-Licht bestrahlt werden.

**13.** Verfahren nach einem oder mehreren der Ansprüche 1 bis 12, dadurch gekennzeichnet, daß der Lösung feinteilige Aktivkohle beigemischt wird.

Europäisches
Patentamt

**EUROPÄISCHER
RECHERCHENBERICHT**

Nummer der Anmeldung

**EP 91 25 0201**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DATABASE WPIL Derwent Publications Ltd., London, GB; DATABASE WPIL, accession no. 86-207843 [32], Woche 8637; & JP - A - 61139385 (S. HASHIMOTO) 26.06.1986 * Zusammenfassung * — — — | 1,5,7 | C 12 N 11/04 |
| X | DATABASE WPIL Derwent Publications Ltd., London, GB; DATABASE WPIL, accession no. 88-166506 [24], Woche 8824; & SU - A - 1351974 (HETEROORG CPDS INST) 15.11.1987 * Zusammenfassung * — — — | 1 | |
| D,Y | EP-A-0 060 052   (RESEARCH ASSOCIATION FOR PE-TROLEUM ALTERNATIVE DEVELOPMENT) * insgesamt * — — — | 1-5,8 | |
| Y | CHEMICAL ABSTRACTS Band 104, Nr. 6, 10. Februar 1986, Seite 25, Zusammenfassung Nr. 34695m, Columbus, Ohio, US; & JP - A - 60177066 (BIOMATERIAL UNIVERS K.K. et al.) 11.09.1985 * Zusammenfassung * — — — | 1-5,8 | RECHERCHIERTE SACHGEBIETE (Int. Cl.5) |
| A | EP-A-0 303 122   (KURARAY CO. LTD.) * insgesamt * — — — | 1,5,7 | C 12 N 11/04 C 12 N 11/02 C 12 N 11/00 |
| A | PATENT ABSTRACTS OF JAPAN Band 13, Nr. 101 (C-574)(3449), 9. März 1989; & JP - A - 63276488 (MITSUBISHI HEAVY IND. LTD.) 14.11.1988 * Zusammenfassung * — — — | 1,3,5 | |

–/–

Der vorliegende Recherchenbericht wurde für alle Patentansprüche erstellt

| Recherchenort | Abschlußdatum der Recherche | Prüfer |
|---|---|---|
| Berlin | 14 November 91 | JULIA P. |

**Europäisches Patentamt**

# EUROPÄISCHER RECHERCHENBERICHT

Nummer der Anmeldung

## EP 91 25 0201

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DATABASE WPIL Derwent Publications Ltd., London, GB; DATABASE WPIL, accession no. 88-094872 [14], Woche 8814; & JP - A - 63044994 (MITSUBISHI HEAVY IND KK) 25.02.1988 * Zusammenfassung * — — — | 1 | |
| A | DATABASE WPIL Derwent Publications Ltd., London, GB; DATABASE WPIL, accession no. 88-267801 [38], Woche 8838; & JP - A - 63195438 (TOYOTA JIDOSHA KK) 12.08.1988 * Zusammenfassung * — — — | 1,6 | |
| A | DATABASE WPIL Derwent Publications Ltd., London, GB; DATABASE WPIL, accession no. 89-265378 [37], Woche 8942; & JP - A - 012228454 (R & D ASSOC BIOREAC et al.) 12.09.1989 * Zusammenfassung * — — — | 11,12 | |
| A | US-A-4 727 030   (F. ISHIMURA et al.) * insgesamt, insbesondere Seite 5, Zeilen 45-56, Seite 7, Zeilen 20-32 * — — — | 1,13 | RECHERCHIERTE SACHGEBIETE (Int. Cl.5) |
| A | FR-A-2 599 639   (RAMOT UNIVERSITY FOR APPLIED RESEARCH AND INDUSTRIAL DEVELOPMENT LTD.) — — — | 1,10 | |
| A | DATABASE WPIL Derwent Publications Ltd., London, GB; DATABASE WPIL, accession no. 86-166560 [26]; & JP - A - 61100193 (S. HASHIMOTO) 05.05.1986 * Zusammenfassung * — — — | 1,3,5,7 | |

−/−

Der vorliegende Recherchenbericht wurde für alle Patentansprüche erstellt

| Recherchenort | Abschlußdatum der Recherche | Prüfer |
|---|---|---|
| Berlin | 14 November 91 | JULIA P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
    nach dem Anmeldedatum veröffentlicht worden ist
D : in der Anmeldung angeführtes Dokument
L : aus anderen Gründen angeführtes Dokument
‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑‑
& : Mitglied der gleichen Patentfamilie,
    übereinstimmendes Dokument

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | BIOTECHNOLOGY ABSTRACTS Derwent Publications Ltd., London, GB; accession no. 91-04182 & V.L. LOZINSKY et al.: "Application of poly(vinyl alcohol) cryogels in biotechnology" & Biotekhnologiya 1990, Band 5, Seiten 32-35 * Zusammenfassung * — — — | 1-3 | |
| A | BIOTECHNOLOGY ABSTRACTS Derwent Publications Ltd., London, GB; accession no. 88-03653 & O. ARIGA et al.: "Immobilization of Microorganisms with PVA hardened by iterative freezing and thawing" & J. Ferment. Technol. 1987, Band 65, Nr. 6, Seiten 651-658 — — — — — | 1-3 | |

**RECHERCHIERTE SACHGEBIETE (Int. Cl.5)**

Der vorliegende Recherchenbericht wurde für alle Patentansprüche erstellt

| Recherchenort | Abschlußdatum der Recherche | Prüfer |
|---|---|---|
| Berlin | 14 November 91 | JULIA P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

-------------------------------------------------------------------

& : Mitglied der gleichen Patentfamilie, übereinstimmendes Dokument